# FASCICULE DE BREVET EUROPEEN

(11) **EP 2 288 341 B1**
(45) Date de publication et mention de la délivrance du brevet: **21.06.2017**
(21) Numéro de dépôt: 09761874.8
(22) Date de dépôt: 20.05.2009
(51) Int. Cl.: A61K 9/20, A61K 9/28, A61K 33/06, A61K 9/70

(54) **UTILISATION D'UNE MATRICE POUR ADMINISTRATION ORALE DE MAGNESIUM A LIBERATION PROLONGEE, ET COMPOSITION CONTENANT CETTE MATRICE**
VERWENDUNG EINER MATRIX ZUR ORALEN VERABREICHUNG VON MAGNESIUM MIT VERZÖGERTER FREISETZUNG UND DIESE MATRIX ENTHALTENDE ZUSAMMENSETZUNG
USE OF A MATRIX FOR ORALLY ADMINISTERING SUSTAINED RELEASE MAGNESIUM, AND COMPOSITION CONTAINING SAID MATRIX

(30) Priorité: 20.05.2008 FR 0802702
(43) Date de publication de la demande: 02.03.2011
(73) Titulaire: Joanny, Fabienne, 75008 Paris (FR)
(72) Inventeur: Joanny, Fabienne, 75008 Paris (FR)
(74) Mandataire: Tezier Herman, Béatrice
(86) Numéro de dépôt international: PCT/FR2009/000585
(87) Numéro de publication internationale: WO 2009/150323

(56) Documents cités:
- US-A1- 2004 156 896
- US-A1- 2005 220 865
- US-A1- 2005 266 082
- US-A1- 2006 217 385

## Description

### Domaine de l'invention

La présente invention a trait à une nouvelle utilisation d'une matrice pour administration orale de magnésium sous la forme comprimé à libération prolongée, afin d'améliorer la biodisponibilité du magnésium par le biais d'une dissolution progressive et continue tout le long du tractus gastro-intestinal. Elle concerne également un produit industriel nouveau qui est une composition contenant du magnésium à libération prolongée, notamment en tant que complément alimentaire, et se présentant sous la forme de comprimé.

### Art antérieur

On connait des demandes de brevet US 2005/220865, US 2006/217385, US 2005/266082 et US 2004/156896 des compositions pharmaceutiques orales, en général des comprimés, qui comprennent les substances suivantes : magnésium ou dérivé de magnésium, hydroxypropylcellulose, lactose, silice colloïdale et béhénate de glycéryle choisi dans une liste d'agents possibles. Aucun de ces documents ne prévoient toutefois des compositions à administration orale comprenant l'ensemble de ces agents aux quantités spécifiées selon l'invention.

On sait de l'ouvrage de BERTHELOT A., ARNAUD M., et REBA A., 'Le magnésium' pages 27-30, éditeur John Libbey Eurotext (collection 'Pathologie Science Formation'), décembre 2004, (i) qu'il est difficile de doser le magnésium de l'organisme ; (ii) que pour évaluer le métabolisme, on mesure par commodité le taux de magnésium plasmatique, celui-ci n'étant cependant pas toujours un bon reflet du pool magnésique global, car des teneurs d'environ 12 % à 23 % en poids du magnésium corporel sont rapidement échangeables, notamment avec le sodium, le potassium et le calcium ; et (iii) que la région de l'iléon est le site le plus favorable pour le passage du magnésium à travers la paroi intestinale.

On sait notamment de l'article de ROTH P. et WERNER E., 'Intestinal Absorption of Magnésium in Man', International Journal of Applied Radiation and Isotopes, 1979:30, 523-526, que, par administration orale de l'isotope ²⁸Mg chez l'homme, la biodisponibilité, exprimée sous la forme de pourcentage de magnésium absorbé par rapport à la quantité pondérale de magnésium administré, décroît quand la dose de magnésium augmente. Voir à ce sujet le Tableau 1 de cet article, qui est résumé ci-après :

**Résumé du Tableau 1 de ROTH P. et al.**

| Dose orale de Mg (mmol) | Fraction moyenne absorbée (%) |
|---|---|
| 0,3 | 0,70 |
| 1,3 | 0,48 |
| 4,2 | 0,29 |
| 12,5 | 0,20 |
| 41,7 | 0,14 |

On sait, notamment des publications WO 01/22943 A, US 6887492 B, US 5849338 A et GB 1356097 A illustrant l'état antérieur de la technique, que l'on a déjà proposé dans le passé des solutions techniques destinées à fournir du magnésium retard (i. e. à libération décalée, lente ou réduite dans le temps).

Le brevet GB 1356097 A propose une composition de polyélectrolytes à libération lente ('slow release') pour la substitution continue de sels minéraux perdus lors de surcharges corporelles, de forte transpiration et d'utilisation de diurétiques. Cette composition se présente sous la forme d'une matrice contenant sous la forme de sels 5 à 80 parties en poids de Ca, 5 à 40 parties en poids de Mg, 2 à 20 parties en poids de K, pas de Na ou moins de 20 parties en poids de Na. Ce brevet ne s'intéresse pas à la libération du magnésium seul, en outre il ne décrit pas les moyens mis en oeuvre pour obtenir la libération prolongée et lente recherchée.

Le brevet US 5849338 A préconise une composition retard pour le traitement de la vasoconstriction, qui contient en association plusieurs ingrédients actifs, notamment l'association :
- de MgO et/ou d'au moins un sel de Mg,
- d'une substance de la famille de la vitamine E,
- d'acide ascorbique ou d'un ascorbate,
- de sélénium,
- d'acide folique ou d'un folate, et
- d'une hormone, la mélatonine ;
et en tant qu'excipient une matrice retard à base de polymères cellulosiques ou de pellicules qui résistent aux acides. Ce brevet ne décrit ni ne suggère les moyens mis en oeuvre selon l'invention pour une libération prolongée et lente du magnésium.

Le brevet US 6887492 B prévoit une composition unitaire, sous forme comprimé ou capsule, comprenant :
- au moins un composé de magnésium libérant Mg au niveau de l'intestin, qui est logé dans un noyau pourvu d'un revêtement entérique, et
- une couche périphérique contenant du calcium libérable au niveau gastrique qui enrobe ledit revêtement entérique.

La présente invention se distingue du brevet US 6887492 B par la dissolution d'une portion du Mg au niveau gastrique, d'une part, et par l'absence de Ca, d'autre part.

La demande internationale publiée WO 01/22943 A propose d'incorporer une substance active hygroscopique [telle que la carnitine (notamment la L-carnitine, la DL-carnitine ou l'acétyl-L-carnitine)], liquide ou gazeuse, dans un ciment magnésien ('ciment de Sorel') pour obtenir une forme retard libérant ladite substance active et du magnésium. Le ciment de Sorel, obtenu à partir de la réaction :

MgCl₂.6H₂O + 5MgO → MgCl₂.5MgO.H_{2O} + 5H₂O

puis déshydratation réduite lors du séchage, est un mélange d'oxychlorures de magnésium de structure :

MgCl₂.aMgO.(6-a)H₂O

où a est un nombre entier ayant pour valeur 1, 2, 3, 4 ou 5, dans ce mélange les composés pondéralement prépondérants étant ceux dans lesquels a = 2 et a = 3. La solution technique du ciment de Sorel semble élégante, cependant elle parait inefficace ou inappropriée car l'invention objet de la demande internationale WO 01/22943 A n'a pas encore été exploitée ou ne semble guère exploitable (en particulier les phases nationales de cette demande internationale n'ont même pas été déclenchées).

Par ailleurs, en ce qui concerne les compositions orales de magnésium à libération prolongée, on connaît le brevet européen délivré EP 0542979 B et surtout la publication de la demande internationale WO 2004/105778 A, ces deux documents constituant l'art antérieur le plus proche de l'invention.

Le brevet EP 0542979 B vise une composition thérapeutique utile vis-à-vis des carences magnésiennes et destinée à assurer une libération prolongée du magnésium, qu'elle contient, sous forme de Mg²⁺ assimilable au niveau entéral afin de compléter jusqu'à au moins 6 mg/kg l'apport quotidien en magnésium chez l'homme, ladite composition étant caractérisée en ce qu'elle comporte un mélange comprenant, en association avec un excipient physiologiquement acceptable,
(a) 4 à 14 parties en poids de magnésium provenant d'une source constituée par MgO, MgCl₂ et les hydrates de formule MgCl₂.n(H₂O) où n est un nombre entier ou fractionnaire supérieur à 0 et inférieur ou égal à 6,
(b) 6 à 13 parties en poids d'un polymère hydrophile, quand la source de Mg est MgO, ou 10 à 30 parties en poids d'une substance hydrophobe choisie parmi l'ensemble constitué par les polymères hydrophobes physiologiquement acceptables, les esters d'acides gras et leurs mélanges, quand la source de Mg est différente de MgO, et
(c) 6 à 16 parties en poids d'une charge inerte intervenant en tant que diluant solide et notamment choisie parmi l'ensemble constitué par le lactose, les phosphates de métaux alcalins et alcalino-terreux et leurs mélanges,
la teneur en Mg dans ledit mélange étant comprise entre 5 et 60 % en poids par rapport au poids dudit mélange.

La composition selon EP 0542979 B est généralement enrobée dans une enveloppe, qui est un pelliculage gastrorésistant, et se présente sous la forme d'un comprimé contenant 50 mg de magnésium et destiné à une ou deux prises quotidiennes.

La demande internationale publiée WO 2004/105778 A propose une combinaison utile en cosmétique, en thérapeutique et/ou dans le domaine de la nutrition, pour agir notamment contre les états de stress, caractérisée en ce qu'elle consiste en
(α) une préparation (I) de magnésium à libération prolongée comprenant, en association avec un excipient physiologiquement acceptable un mélange :
   (A) de magnésium provenant d'une source de magnésium constituée par MgO, MgCl₂ et les hydrates de formule MgCl₂.n(H₂O) où n est un nombre entier ou fractionnaire supérieur à 0 et inférieur ou égal à 6,
   (B) d'au moins une substance choisie parmi B1 et B2 ou une de leurs associations :
      (B1) un polymère hydrophile, qui est un dérivé de cellulose, et/ou
      (B2) une substance hydrophobe faisant partie de la famille des esters d'acides gras avec des polyols, et
   (C) d'une charge inerte intervenant en tant que diluant solide, notamment le lactose,
   la teneur en Mg dans ledit mélange étant comprise entre 1 et 60 % en poids par rapport au poids dudit mélange A + B + C ; et,
(β) une substance active (Z) qui est notamment un extrait de plante (tel que valériane, mélisse, thym maritime, écorce de pin maritime, aubier de tilleul, céréale, pomme, melon) ou d'algue.

La préparation I et la substance Z peuvent être administrées par voie orale, soit simultanément sous un même conditionnement, soit séparément (notamment dans le cadre d'une polythérapie) sous deux conditionnements distincts.

L'enrobage de la préparation I ou celui du conditionnement unique de l'ensemble I + Z est réalisé au moyen d'un pelliculage gastrorésistant. Sous la forme d'un comprimé unitaire (contenant 100 mg de magnésium) pour une seule prise quotidienne, l'on administre soit la composition I dans le cadre d'une polythérapie, soit le mélange de I et Z dans le cadre d'une réalisation galénique unique.

La composition avec enrobage gastrorésistant selon EP 0542979 B et la préparation I également avec enrobage gastrorésistant selon WO 2004/105778 A donnent de bons résultats en ce qui concerne la libération prolongée du magnésium à partir d'un comprimé. Cependant, ladite composition selon EP 0542979 B et ladite préparation I selon WO 2004/105778 A ne sont guère satisfaisantes sur le plan de la biodisponibilité du magnésium.

### But de l'invention

Il existe un besoin d'améliorer la biodisponibilité du magnésium à libération prolongée (en anglais : «sustained release») dans le temps. En particulier, quand on apprécie la cinétique de la libération du magnésium *in vitro* au moyen du système classique :
- dissolution avec un milieu acide [avantageusement 900 ml selon la pharmacopée américaine] HCl 0,1N de l'instant T = 0 à l'instant T = 2h (i. e. traitement correspondant approximativement à la durée de transit dans l'estomac), puis
- dissolution dans un tampon [avantageusement 900 ml] à pH 6,8 de l'instant T = 2h à T = 8h [i. e. traitement correspondant approximativement à la durée de transit dans l'intestin grêle (de T = 2h à T = 4h) puis traitement correspondant approximativement au transit dans le gros intestin (de T = 4h à T = 8h)],
on constate que, lorsque l'on utilise un enrobage gastrorésistant selon la composition du brevet EP 0542979 B ou selon la préparation I de la demande publiée WO 2004/105778 A, la cinétique de libération de Mg²⁺ (rapport : teneur en % en poids de Mg dissous/temps), qui est substantiellement nulle de T = 0 à T = 2h, est trop forte dans l'intervalle de T = 2h à T = 4h, ce qui diminue la biodisponibilité.

On a aussi constaté de façon surprenante selon la présente invention, qu'il fallait que la dissolution
(i) commence dans la phase 'gastrique' (de T = 0 à T = 2h) avec une cinétique de dissolution ralentie [le taux de dissolution (δ) du magnésium par rapport au magnésium administré par le biais de la source de magnésium] étant inférieur ou égal à 60 %], pour que :
(ii) Mg arrive à dose filée au niveau de l'intestin grêle où il commence à être absorbé avec une cinétique faible (de T = 2h à T = 4h), d'une part, puis
(iii) Mg arrive dans la phase 'gros intestin' (de T =4h à T = 8h), d'autre part.

L'invention repose en particulier sur (1°) l'absorption préférentielle de Mg²⁺ au niveau de l'iléon, lieu où l'absorption du Mg est maximale, et (2°) une dissolution plus lente et progressive, qui est programmée, de la sortie de l'estomac jusqu'au gros intestin.

Selon la présente invention, la libération du magnésium sous la forme Mg²⁺ intervient en continu dans tout le tractus gastro-intestinal de l'estomac au gros intestin, alors que l'absorption du magnésium (toujours sous la forme Mg²⁺) se fait tout le long du tractus intestinal du duodénum au gros intestin, l'absorption étant maximale au niveau de l'iléon (i. e. la dernière partie de l'intestin grêle).

Selon la présente invention, on se propose de fournir une nouvelle composition de formulation, qui est certes incluse dans la famille de la préparation I de WO 2004/105778 A, mais non encore décrite, et qui procure une biodisponibilité améliorée, afin de satisfaire le besoin précité.

### Objet de l'invention

Selon un premier aspect de l'invention, on propose l'utilisation d'une matrice pour la préparation d'une composition pour l'administration orale, sous la forme de comprimé, de magnésium avec libération prolongée, qui est dépourvue d'un enrobage gastrorésistant mais pourvue d'un enrobage de protection ralentissant ou freinant la dissolution du Mg au niveau gastrique, ladite matrice, qui forme un noyau constitué d'un agent de retard hydrophile (B1), d'un agent de retard hydrophobe (B2), d'une charge inerte (C1) intervenant en tant que diluant et d'une charge inerte (C2) intervenant en tant que moyen lubrifiant, étant caractérisée en ce qu'elle comprend, pour administrer
(A) 90 à 110 parties en poids de magnésium,
les ingrédients suivants :
(B1) 180 à 190 parties en poids d'hydroxypropylméthylcellulose,
(B2) 19,8 à 22,2 parties en poids de béhénate de glycéryle,
(C1) 10 à 12 parties en poids de lactose, et
(C2) 10 à 12 parties en poids de silice colloïdale.

L'enrobage de la matrice (i. e. l'enrobage du comprimé nu) n'est pas gastrorésistant. Il intervient pour la protéger, notamment au cours du conditionnement et du stockage, d'une part, et il sert à ralentir la libération du magnésium au niveau gastrique, d'autre part. Il représente en général 15 à 75 parties en poids pour une quantité de 90 à 110 parties en poids de Mg (i. e. approximativement 1,3 à 7,5 % en poids par rapport au poids de la matrice, c'est-à-dire du comprimé nu).

On a déterminé *in vitro* le taux de dissolution (δ) du magnésium (exprimé en % par rapport au magnésium total apporté par la source de magnésium). La matrice enrobée selon l'invention fournit, après 2h en milieu HCl 0,1N, un taux de dissolution δ qui est inférieur ou égal à 60 %.

Quand on souhaite apprécier la cinétique globale de dissolution *in vitro* d'un comprimé, on met en oeuvre un système de dissolution classique (désigné ici «système de dissolution A») d'abord dans un milieu HCl 0,1N de T = 0 à T = 2h, puis dans un tampon à pH 6,8 de T = 2h à T = 8h, afin de déterminer les teneurs cumulées en substance active dissoute, ici le magnésium, aux instants T = 2h, T = 4h, T = 6h et T = 8h. Cette cinétique de dissolution est déterminée à une température pouvant se situer de la température ambiante (15-25 °C) jusqu'à 40 °C. Comme dans la présente invention le comprimé enrobé et ses constituants sont tous stables au stockage pendant plusieurs mois à 40 °C, la cinétique de dissolution a été mesurée ici à 40 °C par commodité pour se placer dans des conditions de température sensiblement proches de celle de l'intérieur du corps humain.

Le comprimé enrobé selon l'invention présente un profil de dissolution tel que :
- à T = 2h, on a : δ ≤, 60 %, plus précisément : 20 % ≤ δ ≤ 60 %, et de préférence : 25 % ≤ δ ≤ 58 % ;
- à T = 4h, on a : δ ≤ 85 %, plus précisément : 40 % ≤ δ ≤ 85 %, et de préférence : 45 % ≤ δ ≤ 82 % ;
- à T = 6h, on a : δ ≤ 98 %, plus précisément : 60 % ≤ δ ≤ 98 %, et de préférence : 80 % ≤ δ ≤ 95 % ; et
- à T = 8h, on a : δ ≤ 100 %, plus précisément : 90 % ≤ δ ≤ 100 %, et de préférence : 95 % ≤ δ ≤ 99,9 %.

Selon un autre aspect de l'invention, on propose pour administration orale de magnésium, sous forme de comprimé, avec libération prolongée une composition caractérisée en ce qu'elle est constituée
- d'une matrice formant un noyau comprenant en mélange :
   (A) une quantité de MgCl₂.9/2H₂O fournissant 90 à 110 parties en poids de magnésium
   (B1) 180 à 190 parties en poids d'hydroxypropylméthylcellulose,
   (B2) 19,8 à 22,2 parties en poids de béhénate de glycéryle,
   (C1) 10 à 12 parties en poids de lactose, et
   (C2) 10 à 12 parties en poids de silice colloïdale ; et,
- d'un enrobage de protection ralentissant la libération du magnésium au niveau gastrique, qui n'est pas gastrorésistant.

Selon encore un autre aspect, on fournit une nouvelle utilisation d'une composition selon l'invention, caractérisée en ce que l'on fait appel à une dite composition pour la préparation d'une spécialité se présentant sous la forme comprimé et destinée à un usage du magnésium à libération prolongée en tant que complément alimentaire.

### Description détaillée de l'invention

Comme l'absorption du magnésium se fait tout le long du tractus intestinal, du duodénum au gros intestin, et est optimale au niveau de l'iléon (i. e. la dernière partie de l'intestin grêle), la durée du transit variant avec le type de repas, et comme dans le système de dissolution A précité à T = 2h il reste du Mg administré non encore dissous, une quantité optimalement absorbable du magnésium libéré (sous la forme Mg²⁺) parvient selon l'invention au niveau de l'iléon pour traverser la paroi intestinale.

On a selon l'invention une cinétique de dissolution selon laquelle la libération de Mg intervient de façon (i) relativement lente et (ii) progressive dès la phase 'gastrique'. Cette cinétique, déterminée au moyen du système de dissolution A, est donnée dans le Tableau I produit ci-après pour des comprimés selon l'invention (Ex 1-Ex 5, Ex 7 et Ex 11-Ex 12) et des comprimés comparatifs (CP 1 à CP 5). Elle implique une dissolution particulière au niveau de l'estomac (ce qui interdit un pelliculage gastrorésistant), d'une part, et au niveau de l'intestin grêle, d'autre part.

Les quantités pondérales A, B1, B2, C1 et C2 données plus haut sont celles qui fournissent la biodisponibilité optimale en magnésium. Elles fournissent à l'issue de la phase 'intestin grêle' (i. e. à T = 4h), à 40 °C, une teneur en magnésium dissous généralement plus faible ou analogue à celle de l'art antérieur. A la différence de l'art antérieur, le profil de dissolution de la matrice enrobée selon l'invention présente une cinétique ayant une pente relativement faible dans la phase 'intestin grêle' (voir notamment la cinétique de Ex 11 et Ex 12 dans ledit Tableau I)

La matrice selon l'invention et son enrobage ne comportent aucun produit prohibé par les réglementations européennes et internationales relatives aux compléments alimentaires. En particulier, ladite matrice et ledit enrobage sont dépourvus de PCV et de polyvinylpyrrolidone.

La quantité de Mg dissous à T = 4h (40 à 85 % du Mg administré et de préférence 45 à 82 % du Mg administré) est importante pour obtenir une matrice qui convienne pour (i) la compression, (ii) la libération progressive et continue que l'on recherche et surtout (iii) une biodisponibilité optimale du magnésium. Cette quantité conduit à une libération convenable au niveau de l'iléon, d'une part, et conduit également, en relation avec la quantité de Mg dissous dans la phase 'gastrique', à une libération plus lente et progressive, ce qui favorise une absorption mieux adaptée au mécanisme physiologique du passage du magnésium à travers la paroi intestinale, d'autre part.

La substance B1 est l'hydroxypropylméthylcellulose (HPMC). Elle est utilisée ici suivant une qualité qui convient pour usage pharmaceutique ou alimentaire.

La substance B2 est le béhénate de glycéryle, qui est un mélange essentiellement constitué de monoglycéride et de diglycéride de l'acide béhénique (autre nomenclature: 'mono-diglycéride béhénate') et connu sous la dénomination européenne d'additif 'E471'. La substance B2 est également utilisée ici suivant une qualité qui convient pour usage pharmaceutique ou alimentaire.

Selon l'invention, le rapport pondéral B1/B2 est compris entre 180/22,2 = 8,1/1 et 190/19.8 = 9,6/1. Avantageusement, on recommande que ledit rapport pondéral soit compris entre 8,5/1 et 9,3/1. De préférence, le rapport pondéral B1/B2 se situera entre 8,7/1 et 9,2/1, par exemple : 8,8/1 ou 9/1 ou encore 9,15/1.

Le lactose, composant C1, est avantageusement anhydre. De même la silice colloïdale, composant C2, est avantageusement anhydre. En pratique, il est plutôt préféré que dans la matrice de l'invention le rapport pondéral C1/C2 soit voisin de 1/1 et mieux égal à 1/1.

L'enrobage de l'invention n'est pas gastrorésistant. Il s'agit d'un pelliculage qui intervient (i) pour protéger les composants du comprimé nu vis-à-vis de l'extérieur, notamment vis-à-vis des chocs, et surtout (ii) pour ralentir la dissolution du Mg dans la phase 'gastrique'. Ce pelliculage peut être réalisé en une seule couche, deux couches, voire même trois couches. Pour limiter les coûts de fabrication, il est possible qu'il soit monocouche. Toutefois, l'on recommande avantageusement un enrobage à deux couches pour mieux contrôler la dissolution du Mg. Comme indiqué plus haut, l'enrobage de la matrice représente en général 15 à 75 parties en poids pour une source de magnésium fournissant 90 à 110 parties en poids de Mg (i. e. approximativement 1,3 à 7,5 % en poids par rapport au poids de la matrice). De préférence il représentera 15 à 70 parties en poids, et mieux 15 à 45 parties en poids, pour 90 à 110 parties en poids de Mg.

Les substances recommandées ici pour l'enrobage sont la gomme laque, et les éthers de cellulose filmogènes tels que les alkylcelluloses, à savoir plus particulièrement les mélanges de HPMC et d'hydroxypropylcellulose (HPC) commercialisés notamment sous les nomenclatures de NUTRATERIC^{®} et OPADRY^{®}. On peut également envisager un enrobage constitué d'une première couche de gomme laque et d'une couche externe faite d'un mélange d'alkylcelluloses.

En pratique, on préconise un enrobage qui est
(a) un pelliculage monocouche de gomme laque (utilisée à 50 % en poids dans de l'éthanol, le solvant étant éliminé lors du pelliculage), ou
(b) un pelliculage à deux couches, chaque couche comprenant une substance choisie parmi la gomme laque, les éthers de cellulose (notamment HPMC et HPC) et leurs mélanges.

Quand on utilise un enrobage à deux couches, la première couche (ou couche interne) représente en général 0.5 à 4 % en poids par rapport au poids de la matrice, et la seconde couche (ou couche externe) représente en général 0,5 à 3.5 % en poids par rapport au poids de ladite matrice, l'ensemble des deux dites couches représentant 1,3 à 7,5 % en poids par rapport au poids de ladite matrice.

La source de Mg est selon l'invention constituée par MgO, MgCl₂ et les hydrates de formule MgCl₂.n(H₂O) où n est un nombre entier ou fractionnaire supérieur à 0 et inférieur ou égal à 6. Les sels de Mg avec les acides organiques ne conviennent généralement pas ici (notamment quand il s'agit de sels d'acides gras). En effet, (a) le pourcentage pondéral du magnésium dans ces sels diminue lorsque la masse moléculaire augmente, et (b) par suite ces sels conduisent à des comprimés de taille et masse si importants qu'il devient difficile de les avaler. La source préférée selon l'invention est un hydrate, à savoir MgCl₂.9/2H₂O.

Selon l'invention, on préconise une composition, sous la forme de comprimé pelliculé, qui libère du magnésium de façon progressive et continue. Cette composition est avantageusement constituée :
- d'une matrice constituant un noyau comprenant
   (A) 90 à 110 parties en poids de magnésium, la source de magnésium étant MgCl₂.9/2H₂O
   (B1) 180 à 190 parties en poids d'hydroxypropylméthylcellulose,
   (B2) 19,8 à 22,2 parties en poids de béhénate de glycéryle,
   (C1) 10 à 12 parties en poids de lactose, et
   (C2) 10 à 12 parties en poids de silice colloïdale ; et
- d'un pelliculage de
   (D) 15 à 45 parties en poids d'une substance choisie parmi la gomme laque, les éthers de cellulose (notamment HPMC et HPC) et leurs mélanges.

On recommande de conserver la composition selon l'invention à une température inférieure à 40 °C, et de préférence à une température inférieure ou égale à 25 °C.

Cette composition est particulièrement utile en tant que complément alimentaire. Elle intervient en outre avantageusement en tant que cosmétique pour (i) l'hydratation de la peau, et/ou (ii) traiter ou prévenir le stress de la peau. L'intérêt cosmétique du Mg apporté par cette composition au niveau de la peau peut être apprécié par :
- la mesure de l'impédance électrique de la peau (exprimée en Ω) ou son inverse, la conductance (exprimée en S), le taux d'hydratation de la peau étant inversement proportionnel à l'impédance et proportionnel à la conductance, voir à cet effet les méthodes décrites par KALIA Y. et al., Biophys. J. 1996;71(5):2692-2700, KALIA Y. et al., J. Pharm. Sci. 1998;87(12):1508-1811*,* CURDY C. et al., AAPS Pharm. Sci. 2000;2(3):E23, et CLAR E. J.et al., J. Cosm. Chem. 1975;26:337-357 ; et/ou
- l'analyse d'explants de peau humaine maintenus en survie.

Par ailleurs, il est signalé que la demande internationale parente, déposée le même jour que la présente demande et intitulée *'Système à base de magnésium et son utilisation en cosmétique'* vise une utilisation particulière de ladite composition en cosmétique.

D'autres avantages et caractéristiques de l'invention seront mieux compris à la lecture qui va suivre d'exemples de réalisation ('Ex') et d'exemples comparatifs ('CP'), d'une part, et de résultats d'essais comparatifs, d'autre part. Bien entendu l'ensemble de ces éléments n'est pas limitatif mais est donné à titre d'illustration.

Les essais relatifs à détermination de la cinétique de dissolution du Mg ont été réalisés à 40 °C *in vitro* au moyen du système A précité : milieu HCl 0,1N de T = 0 à T = 2h, puis milieu tampon à pH 6,8 de T = 2h à T = 8h.

### Exemple 1

On a préparé des comprimés (dosés à 100 mg en magnésium) ayant la formulation suivante (le rapport pondéral B1/B2 étant de 9,15/1), Qté/cp représentant la quantité (exprimée en mg) de chaque constituant du comprimé.

| Constituants | Qté/cp (mg) |
|---|---|
| *Noyau* : | |
| MgCl₂.9/2H₂O | 725,0 |
| HPMC | 183,0 |
| Mono-diglycéride béhénate | 20,0 |
| Lactose anhydre | 11,0 |
| Silice colloïdale anhydre | 11,0 |

| *Pelliculage :* | |
|---|---|
| Gomme laque | 39,6 |
| *Total* : | 989,60 |

Pour le profil de dissolution de Ex 1, voir Tableau I ci-après.

### Exemple 2

On a préparé des comprimés (dosés à 100 mg en magnésium) ayant la formulation suivante (le rapport pondéral B1/B2 étant de 9,35/1).

| Constituants | Qté/cp (mg) |
|---|---|
| *Noyau* : | |
| MgCl₂.9/2H₂O | 725,0 |
| HPMC | 185,2 |

| | |
|---|---|
| Mono-diglycéride béhénate | 19,8 |
| Lactose anhydre | 11,0 |
| Silice colloïdale anhydre | 11,0 |

| *Pelliculage* : | |
|---|---|
| Gomme laque | 39,42 |
| Bleu patenté | 0,03 |
| *Total* : | 992,45 |

Pour le profil de dissolution de Ex 2, voir Tableau I ci-après.

### Exemple 3

On a préparé des comprimés (dosés à 100 mg en magnésium) ayant la formulation suivante (le rapport pondéral B1/B2 étant de 9,25/1).

| Constituants | Qté/cp (mg) |
|---|---|
| *Noyau* : | |
| MgCl₂.9/2H₂O | 725,0 |
| HPMC | 185,0 |
| Mono-diglycéride béhénate | 20,0 |
| Lactose anhydre | 11,0 |
| Silice colloïdale anhydre | 11,0 |

| *Pelliculage* : | |
|---|---|
| Gomme laque | 39,6 |
| *Total* : | 991,60 |

Pour le profil de dissolution de Ex 3, voir Tableau I ci-après.

### Exemple 4

On a préparé des comprimés (dosés à 100 mg en magnésium) ayant la formulation suivante, selon les modalités précitées (le rapport pondéral B1/B2 étant de 9,15/1).

| Constituants | Qté/cp (mg) |
|---|---|
| *Noyau* : | |
| MgCl₂.9/2H₂O | 725,0 |
| HPMC | 183,0 |
| Mono-diglycéride béhénate | 20,0 |
| Lactose anhydre | 11,0 |
| Silice colloïdale anhydre | 11,0 |

| *Pelliculage 1* : | |
|---|---|
| Gomme laque | 24,17 |

| *Pelliculage 2* : | |
|---|---|
| mélange HPMC/HPC 1/3 p/p | 17,514 |
| Bleu patenté | 0,016 |
| *Total* : | 991,70 |

Pour le profil de dissolution de Ex 4, voir Tableau I ci-après.

### Exemple 5

On a préparé des comprimés (dosés à 100 mg en magnésium) ayant la formulation suivante (le rapport pondéral B1/B2 étant de 9,15/1).

| Constituants | Qté/cp (mg) |
|---|---|
| *Noyau* : | |
| MgCl₂.9/2H₂O | 725,0 |
| HPMC | 183,0 |
| Mono-diglycéride béhénate | 20,0 |
| Lactose anhydre | 11,0 |
| Silice colloïdale anhydre | 11,0 |

| *Pelliculage :* | |
|---|---|
| *1^{ère} couche* : Gomme laque | 19,8 |
| *2^{ème} couche (externe)* : Mélange HPMC/HPC 1/4 p/p | 19,8 |
| *Total* : | 989,60 |

Pour le profil de dissolution de Ex 5, voir Tableau I ci-après.

### Exemple 6

On prépare selon les modalités de l'exemple 5, des comprimés renfermant 50 mg de magnésium et ayant pour chaque constituant une quantité qui est la moitié de celle du constituant homologue dudit Ex 5.

### Exemple 7

On a préparé des comprimés (dosés à 100 mg en magnésium) ayant la formulation suivante (le rapport pondéral B1/B2 étant de 9,15/1).

| Constituants | Qté/cp (mg) |
|---|---|
| *Noyau* : | |
| MgCl₂.9/2H₂O | 725,0 |
| HPMC | 183,0 |
| Mono-diglycéride béhénate | 20,0 |
| Lactose anhydre | 11,0 |
| Silice colloïdale anhydre | 11,0 |
| *Pelliculage :* | |

| | |
|---|---|
| *1^{ère} couche (interne)* : HPMC/HPC 1/3 p/p | 19,8 |
| *2^{ème} couche (externe)* : Mélange HPMC/HPC 1/4 p/p | 19,8 |
| *Total* : | 989,60 |

Pour le profil de dissolution de Ex 7, voir Tableau I ci-après.

### Exemple 8

On prépare selon les modalités de l'exemple 7, des comprimés renfermant 50 mg de magnésium et ayant pour chaque constituant une quantité qui est la moitié de celle du constituant homologue dudit Ex 7.

### Exemple 9

On a préparé des comprimés (dosés à 100 mg en magnésium) ayant la formulation suivante (le rapport pondéral B1/B2 étant de 8,8/1).

| Constituants | Qté/cp (mg) |
|---|---|
| *Noyau* : | |
| MgCl₂.9/2H₂O | 725,0 |
| HPMC | 190,0 |
| Mono-diglycéride béhénate | 21,5 |
| Lactose anhydre | 10,0 |
| Silice colloïdale anhydre | 10,0 |
| Chlorhydrate de pyridoxine | 6,0 |

| *Pelliculage* : | |
|---|---|
| Gomme laque | 40,0 |
| *Total* : | 1003,0 |

### Exemple 10

On a préparé des comprimés (dosés à 50 mg en magnésium) ayant la formulation suivante (le rapport pondéral B1/B2 étant de 9,15/1).

| Constituants | Qté/cp (mg) |
|---|---|
| *Noyau* : | |
| MgCl₂.9/2H₂O | 362,50 |
| HPMC | 91,50 |
| Mono-diglycéride béhénate | 10,00 |
| Lactose anhydre | 5,50 |
| Silice colloïdale anhydre | 5,50 |

| *Pelliculage 1* : | |
|---|---|
| Gomme laque (OPAGLOS^{®} NA715G, produit commercialisé par la Sté dite COLORCON) | 1,3 à 2,2 % * |

| *Pelliculage 2* : | |
|---|---|
| mélange HPMC/HPC 1/3 p/p (OPADRY^{®} VMS, produit commercialisé par la Sté dite COLORCON) | 1,1 à 1,6 % * |
| Jaune 20A38069 | 0,008 |

| | |
|---|---|
| Note (*) : pourcentage en poids par rapport au poids du comprimé nu. | |

### Exemple 11

On a préparé des comprimés (dosés à 50 mg en magnésium) ayant la formulation suivante (le rapport pondéral B1/B2 étant de 9,15/1).

| Constituants | Qté/cp (mg) |
|---|---|
| *Noyau* : | |
| MgCl₂.9/2H₂O | 362,50 |
| HPMC | 91,50 |
| Mono-diglycéride béhénate | 10,00 |
| Lactose anhydre | 5,50 |
| Silice colloïdale anhydre | 5,50 |

| *Pelliculage* : | |
|---|---|
| Gomme laque (OPAGLOS^{®} NA715G, produit commercialisé par la Sté dite COLORCON) | 1,7% * |

| | |
|---|---|
| Note (*) : pourcentage en poids par rapport au poids du comprimé nu. | |

Pour le profil de dissolution de Ex 11, voir Tableau I ci-après.

### Exemple 12

On a préparé des comprimés (dosés à 50 mg en magnésium) ayant la formulation suivante (le rapport pondéral B1/B2 étant de 9,15/1).

| Constituants | Qté/cp (mg) |
|---|---|
| *Noyau* : | |
| MgCl₂.9/2H₂O | 362,50 |
| HPMC | 91,50 |
| Mono-diglycéride béhénate | 10,00 |
| Lactose anhydre | 5,50 |
| Silice colloïdale anhydre | 5,50 |

| *Pelliculage 1* : | |
|---|---|
| Gomme laque (OPAGLOS^{®} NA715G, produit commercialisé par la Sté dite COLORCON) | 1,7 * |
| *Pelliculage 2* : | |
| mélange HPMC/HPC 1/3 p/p (OPADRY^{®} VMS, produit commercialisé par la Sté dite COLORCON) | 0,5 % * |

| | |
|---|---|
| Note (*) : pourcentage en poids par rapport au poids du comprimé nu. | |

Pour le profil de dissolution de Ex 12, voir Tableau I ci-après.

### Exemple comparatif CP 1

Le présent exemple comparatif correspond à une spécialité, conforme au brevet EP 0542979 B, du type comprimé (dosé à 100mg en Mg) à enrobage gastrorésistant, qui a été commercialisée en Belgique en 1999 puis retirée du marché voici peu de temps. Sa composition est la suivante.

| Constituants | Qté/cp (mg) |
|---|---|
| *Noyau* : | |
| MgCl₂.6H₂O | 836,09 |
| HPMC | 120,00 |
| Palmitostéarate de glycérol | 112,28 |
| Lactose anhydre | 52,60 |
| Silice colloïdale hydratée | 13,03 |

| *Pelliculage gastrorésistant* : | |
|---|---|
| Acétophtalate de cellulose | 96,00 |
| Phtalate d'éthyle | 24,00 |
| *Total* : | 1254,00 |

Pour le profil de dissolution de CP 1, voir Tableau I ci-après.

### Exemple comparatif CP 2

On a préparé des comprimés de 990 mg chacun selon la formulation de l'exemple 2 du brevet EP 0542979 B, avec la différence que le pelliculage d'ester d'acide gras gastrorésistant a été remplacé par un pelliculage monocouche de gomme laque comme à l'exemple 1 ci-dessus. La formulation est la suivante :

| Constituants | % (en poids) |
|---|---|
| *Noyau* : | |
| MgCl₂.9/2H₂O | 44,50 |
| Chlorhydrate de pyridoxine | 3,00 |
| Lactose anhydre | 14,78 |
| PVC | 18,40 |
| Silice colloïdale anhydre | 0,92 |

| *Pelliculage* : | |
|---|---|
| Gomme laque | 18,40 |
| *Total* : | 100,00 |

Pour le profil de dissolution de CP 2, voir Tableau I ci-après.

### Exemple comparatif CP 3

On a préparé des comprimés selon la formulation suivante.

| Constituants | Qté/cp (mg) |
|---|---|
| *Noyau* : | |
| MgCl₂.6H₂O | 836,00 |
| HPMC | 102,91 |
| Mono-diglycéride béhénate | 100,00 |
| Lactose anhydre | 50,05 |
| Silice colloïdale anhydre | 11,34 |

| *Pelliculage* : | |
|---|---|
| Gomme laque | 40,0 |
| *Total* : | 1140,00 |

La cinétique de dissolution est (a) pratiquement nulle pendant la phase 'gastrique' et (b) trop rapide (pente trop forte) pendant la phase 'intestin grêle'. En effet 1 % du Mg est dissous à T = 2h, et 90 % du Mg est dissous à T = 4h. Voir Tableau I ci-après.

### Exemple comparatif CP 4

On a préparé des comprimés ayant une masse unitaire de 990 mg, sans pelliculage, selon la formulation suivante.

| Constituants | Qté/cp (mg) |
|---|---|
| *Noyau* : | |
| MgCl₂.9/2H₂O | 725,0 |
| Mono-diglycéride béhénate | 202,91 |
| Lactose anhydre | 31,09 |
| Silice colloïdale anhydre | 11,00 |
| *Total* : | 990,00 |

La cinétique de dissolution est (a) nulle pendant la phase 'gastrique' et (b) trop rapide (pente trop forte) pendant la phase 'intestin grêle'. En effet 0 % du Mg est dissous à T = 2h, et 80 % du Mg est dissous à T = 4h. Voir Tableau I ci-après.

### Exemple comparatif CP 5

On a préparé des comprimés ayant la formulation suivante.

| Constituants | Qté/cp (mg) |
|---|---|
| *Noyau* : | |
| MgCl₂.9/2H₂O | 725,0 |
| HPMC | 153,0 |
| Mono-diglycéride béhénate | 50,0 |
| Lactose anhydre | 11,0 |
| Silice colloïdale anhydre | 11,0 |

| *Pelliculage :* | |
|---|---|
| *1^{ère} couche* : Gomme laque | 19,8 |
| *2^{ème} couche (externe)* : Mélange HPMC/HPC 1/4 p/p | 19,8 |
| *Total* : | 989,60 |

La cinétique de dissolution est trop faible au début : 15 % du Mg présent est dissous à T = 2h. Voir Tableau I ci-après.

### Essais comparatifs I

Des essais comparatifs ont été entrepris pour déterminer le profil de la cinétique de dissolution du Mg contenu dans les comprimés de Ex 1-Ex 5, Ex 7, Ex 11-Ex 12 et CP 1-CP 5. Les résultats obtenus, déjà discutés ci-dessus, ont été consignés dans le Tableau I ci-après.

### Essais comparatifs II

Des essais ont été réalisés sur des lots comprenant chacun 5 sujets humains sains, à raison d'un lot par comprimé à tester. Pour avoir affaire à des lots homogènes où les sujets ont approximativement le même volume de sang, on a choisi des lots de sujets du même sexe, du même age ± 1 an, de même poids corporel ± 1 kg, et de même taille ± 1 cm.

On administre aux lots de sujets un comprimé Ex 1, Ex 5, Ex 7, CP 1, CP 2 ou, respectivement, CP 5, chaque matin pendant 14 jours [l'état d'équilibre ('steady state') est en général atteint au bout de 10 jours].

Le 15^{ème} jour, à l'instant T = 0 on prélève 10 ml du sang de chaque sujet et administre un comprimé Ex 1, Ex 5, Ex 7, CP 1, CP 2 ou, respectivement, CP 5, puis recueille le plasma pour mesurer par spectrométrie d'absorption atomique (SAA) le taux plasmatique en magnésium.

A l'instant T = 8h, on prélève à nouveau 10 ml de sang chez chaque sujet et recueille le plasma pour mesurer par spectrométrie d'absorption atomique le taux plasmatique en magnésium.

Les résultats obtenus sont consignés dans le Tableau II donné ci-après.

Ce dosage de magnésium n'est pas une méthode *a priori* totalement pertinente pour apprécier l'absorption intestinale du magnésium, car Mg²⁺ qui passe la paroi intestinale se fixe préférentiellement sur ou dans d'autres tissus, en particulier les os et les érythrocytes, d'une part, et s'échange avec Na⁺, K⁺ et Ca²⁺, d'autre part. Cette mesure permet cependant de vérifier l'intérêt de la cinétique de dissolution particulière de l'invention. Ledit Tableau II montre en particulier que la variation du niveau plasmatique en magnésium entre T = 0 et T = 8h est de 0,09 - 0,10 mmol/l pour les comprimés selon l'invention Ex 1, Ex 2 et Ex 7, alors qu'elle est de 0,02-0,04 mmol/l pour les comprimés de comparaison CP 1, CP 2 et CP 5.

### Divers

Le mode préféré de mise en oeuvre de l'invention consiste à faire appel aux comprimés des exemples 7, 11 et 12. La posologie quotidienne que l'on préconise en Mg est de 50 mg (une prise le matin de Ex 11 ou Ex 12) ou de 100 mg (une prise le matin de Ex 7 ou une prise le matin et une prise le soir de Ex 11 ou Ex 12).

Comme indiqué dans le document WO 2004/105778 A précité, on peut préconiser des combinaisons de la composition selon l'invention, sous la forme comprimé, avec une autre substance active, soit pour une seule administration unitaire, soit pour une administration séparée du Mg et de l'autre substance active.

En particulier on peut incorporer une substance vitaminique telle que Vit. B3, Vit. B12 et/ou coenzyme Q10 (chaque substance vitaminique étant présente à une dose appropriée) soit dans le noyau du comprimé selon l'invention, qui contient MgCl₂.9/2H₂O, soit dans un autre comprimé dépourvu de Mg. Chez l'homme adulte, lesdites doses appropriées, qui sont généralement préconisées *per os,* sont de 18 mg/j pour la Vit. B3, de 1µm/j pour la Vit. B12 et de 30-40 mg/j pour le coenzyme Q10.

On peut aussi recommander une administration distincte : la composition selon l'invention, sous la forme comprimé, étant administrée par voie orale, et l'autre substance active (qui peut être une source d'ions Zn²⁺, un extrait de plante ou d'algue) étant administrée par voie topique. La dose généralement préconisée *per* os chez l'homme adulte est de 15 mg/j pour le zinc.

En variante, quand il s'agit d'administrer un comprimé contenant du Mg et une autre substance active essentiellement libérable au niveau gastrique, on peut prévoir que cette dernière soit disposée en périphérie du noyau ou logée dans la masse dudit enrobage.

Parmi les extraits de plante qui sont utiles ici, on peut citer les extraits de valériane, mélisse, thym maritime, écorce de pin maritime, céréale, pomme et melon, déjà mentionnés dans la demande précitée WO 2004/105778 A, ainsi que les extraits de maca, grenade, son de riz, argousier et rhodiola. L'extrait de plante peut être ici une huile essentielle, par exemple l'huile essentielle de lavande ou l'huile essentielle de néroli. Par ailleurs, on peut également utiliser des extraits d'algues, par exemple un extrait d'algue Klamath (***Aphanizomenon Flos-Aquae***), utile par voie interne ou externe selon l'invention.

A titre d'illustration, voici quelques exemples de combinaisons ('CB') utilisables, chez l'homme adulte, avec la composition orale de magnésium à libération progressive de l'invention.

### Combinaison CB1

Par voie orale on administre un comprimé analogue à celui de Ex 1 ci-dessus, avec la différence qu'il contient dans la masse du pelliculage 20-30 mg de ZnCl₂, pour le soin et l'entretien de la peau.

### Combinaison CB2

Par voie orale on administre un comprimé selon Ex 5 ci-dessus, d'une part, et un comprimé contenant 30 à 50 mg d'extrait de grenade (fruit du grenadier, ***Punica granatum***), d'autre part, pour usage antioxydant.

### Combinaison CB3

Par voie orale on administre un comprimé selon Ex 6 ci-dessus, d'une part, et un comprimé contenant de l'extrait de maca (***Lepidium meyenii***), d'autre part, pour combattre la fatigue physique après l'effort (notamment chez le sportif) ou la fatigue morale.

### Combinaison CB4

Par voie orale on administre un comprimé selon Ex 1 ci-dessus, d'une part, et une composition topique contenant de l'huile essentielle de lavande, d'autre part, pour un usage en tant que moyen de détente eu égard à l'effet relaxant musculaire produit par la combinaison du magnésium à libération progressive de l'huile essentielle de lavande.

**Tableau I**

| **Cinétique de dissolution** | | | | |
|---|---|---|---|---|
| | Taux de dissolution δ (% cumulé de Mg dissous) | | | |
| | dans HCl 0,1N | dans tampon à pH 6,8 | | |
| | à T = 2h | à T = 4h | à T = 6h | à T = 8h |
| Selon l'invention | 20 % ≤ δ ≤ 60 % | 40 % ≤ δ ≤ 85 % | 60 % ≤ δ ≤ 98 % | 90 % ≤ δ ≤ 100 % |
| Ex 1 | δ = 21 % | δ = 41 % | δ = 82 % | δ = 98 % |
| Ex 2 | δ = 25 % | δ = 45 % | δ = 83 % | δ = 97 % |
| Ex 3 | δ = 30 % | δ = 50 % | δ = 85 % | δ = 98 % |
| Ex 4 | δ = 32 % | δ = 52 % | δ = 85 % | δ = 98 % |
| Ex 5 | δ = 27 % | δ = 46 % | δ = 84 % | δ = 99% |
| Ex 7 | δ = 28 % | δ = 47 % | δ = 85 % | δ = 99 % |
| Ex 11 | δ = 53 % | δ = 82 % | δ = 96 % | δ = 99,9 % |
| Ex 12 | δ = 56 % | δ = 87 % | δ = 96 % | δ = 99,9 % |
| CP 1 | δ = 0 % | δ = 80 % | δ = 92 % | δ = 99 % |
| CP 2 | δ = 1 % | δ = 81 % | δ = 93 % | δ = 99 % |
| CP 3 | δ = 1 % | δ = 90 % | δ = 99 % | δ = 99 % |
| CP 4 | δ = 0 % | δ = 80 % | δ = 91 % | δ = 99 % |
| CP 5 | δ = 15 % | δ = 79 % | δ = 94 % | δ = 99 % |

**Tableau II**

| **Niveaux plasmatiques en magnésium par SAA** | | | | |
|---|---|---|---|---|
| Comprimés | Sexe des sujets | Niveaux plasmatiques en Mg (mmole/l) | | |
| | | à T = 0 | à T = 8h | variation de T = 0 à T = 8h |
| Ex 1 | F | 0,95 | 1,04 | 0,09 |
| Ex 5 | M | 1,00 | 1,10 | 0,10 |
| Ex 7 | M | 1,02 | 1,12 | 0,10 |
| CP 1 | F | 0,92 | 0,94 | 0,02 |
| CP 2 | M | 0,98 | 1,00 | 0,02 |
| CP 5 | M | 0,93 | 0,97 | 0,04 |

## Revendications

1. Utilisation d'une matrice pour la préparation d'une composition pour l'administration orale, sous la forme de comprimé, de magnésium avec libération prolongée, qui est dépourvue d'un enrobage gastrorésistant mais pourvue d'un enrobage de protection ralentissant la dissolution du Mg au niveau gastrique, ladite matrice, qui forme un noyau constitué d'un agent de retard hydrophile (B1), d'un agent de retard hydrophobe (B2), d'une charge inerte (C1) intervenant en tant que diluant et d'une charge inerte (C2) intervenant en tant que moyen lubrifiant, étant **caractérisée en ce qu'**elle comprend, pour administrer
(A) 90 à 110 parties en poids de magnésium, les ingrédients suivants :
(B1) 180 à 190 parties en poids d'hydroxypropylméthylcellulose,
(B2) 19,8 à 22,2 parties en poids de béhénate de glycéryle,
(C1) 10 à 12 parties en poids de lactose, et
(C2) 10 à 12 parties en poids de silice colloïdale.

2. Utilisation selon la revendication 1, **caractérisée en ce que** ledit enrobage est un pelliculage à une ou plusieurs couches.

3. Utilisation selon la revendication 2, **caractérisée en ce que** ledit enrobage représente 1,3 à 7,5 % en poids par rapport au poids de la matrice

4. Utilisation suivant la revendication 3, **caractérisée en ce que** ledit enrobage est
(a) un pelliculage monocouche de gomme laque, ou
(b) un pelliculage à deux couches, chaque couche comprenant une substance choisie parmi la gomme laque, les éthers de cellulose (notamment HPMC et HPC) et leurs mélanges.

5. Composition pour administration orale, sous forme de comprimé, de magnésium avec libération prolongée, **caractérisée en ce qu'**elle est constituée
• d'une matrice constituant un noyau comprenant
(A) 90 à 110 parties en poids de magnésium, la source de magnésium étant MgCl₂.9/2H₂O
(B1) 180 à 190 parties en poids d'hydroxypropylméthylcellulose,
(B2) 19,8 à 22,2 parties en poids de béhénate de glycéryle,
(C1) 10 à 12 parties en poids de lactose, et
(C2) 10 à 12 parties en poids de silice colloïdale ; et
• d'un pelliculage de
(D) 15 à 75 parties et de préférence 15 à 45 parties en poids d'une substance choisie parmi la gomme laque, les éthers de cellulose (notamment HPMC et HPC) et leurs mélanges.

6. Composition selon la revendication 5, **caractérisée en ce que** ledit enrobage est
(a) un pelliculage monocouche de gomme laque, ou
(b) un pelliculage à deux couches, chaque couche comprenant une substance choisie parmi la gomme laque, les éthers de cellulose (notamment HPMC et HPC) et leurs mélanges.

7. Composition selon la revendication 5 ou 6, **caractérisée en ce qu'**elle présente *in vitro*, après 2h en milieu HCl 0,1N, un taux de dissolution (δ) du magnésium, qu'elle contient, inférieur ou égal à 60 %.

8. Composition selon la revendication 5 ou 6, **caractérisée en ce que**, par détermination *in vitro* selon un système de dissolution comprenant d'abord le traitement de ladite composition dans un milieu HCl 0,1N de T = 0 à T = 2h, puis le traitement dans un tampon à pH 6,8 de T = 2h à T = 8h, elle a un taux de dissolution (δ) de Mg par rapport au Mg administré tel que
• à T = 2h, on a : δ ≤, 60 %, plus précisément : 20 % ≤ δ ≤ 60 %, et de préférence : 25 % ≤ δ ≤ 58 % ;
• à T = 4h, on a : δ ≤ 85 %, plus précisément : 40 % ≤ δ ≤ 85 %, et de préférence : 45 % ≤ δ ≤ 82 % ;
• à T = 6h, on a : δ ≤ 98 %, plus précisément : 60 % ≤ δ ≤ 98 %, et de préférence : 80 % ≤ δ ≤ 95 % ; et
• à T = 8h, on a : δ ≤ 100 %, plus précisément : 90 % ≤ δ ≤ 100 %, et de préférence : 95 % ≤ δ ≤ 99,9 %.

9. Composition selon l'une quelconque des revendications 5 à 8 en tant que complément alimentaire.

10. Composition selon l'une quelconque des revendications 5 à 8 en tant que cosmétique notamment pour (i) l'hydratation de la peau, et/ou (ii) traiter ou prévenir le stress de la peau.

## Patentansprüche

1. Verwendung einer Matrix zur Herstellung einer Zusammensetzung für die orale Verabreichung, in Form einer Tablette, von Magnesium mit verzögerter Freisetzung, die ohne magensaftresistenten Überzug ist, aber mit einem schützenden Überzug versehen ist, der die Auflösung des Mg im Magen verzögert, wobei besagte Matrix, die einen Kern bildet, bestehend aus einem hydrophilen Retardierungsmittel (B1), einem hydrophoben Retardierungsmittel (B2), einer inerten Ladung (C1), die als Verdünnungsmittel dient, und einer inerten Ladung (C2), die als Gleitmittel dient, **dadurch gekennzeichnet ist, dass** sie für eine Verabreichung von
(A) 90 bis 110 Gewichtsteilen Magnesium,
die folgenden Bestandteile umfasst:
(B1) 180 bis 190 Gewichtsteile Hydroxypropylmethylcellulose,
(B2) 19,8 bis 22,2 Gewichtsteile Glycerylbehenat,
(C1) 10 bis 12 Gewichtsteile Lactose, und
(C2) 10 bis 12 Gewichtsteile kolloidales Siliciumdioxid.

2. Verwendung gemäß Anspruch 1, **dadurch gekennzeichnet, dass** besagter Überzug eine Beschichtung aus einer oder mehreren Schichten ist.

3. Verwendung gemäß Anspruch 2, **dadurch gekennzeichnet, dass** besagter Überzug 1,3 bis 7,5 Gew.-% bezogen auf das Gewicht der Matrix umfasst.

4. Verwendung nach Anspruch 3, **dadurch gekennzeichnet, dass** besagter Überzug
(a) eine einschichtige Beschichtung aus Schellack ist, oder
(b) eine zweischichtige Beschichtung ist, wobei jede Schicht eine Substanz umfasst, ausgewählt aus Schellack, Celluloseethern (insbesondere HPMC und HPC) und Gemischen davon.

5. Zusammensetzung für eine orale Verabreichung, in Form einer Tablette, von Magnesium mit verzögerter Freisetzung, **dadurch gekennzeichnet, dass** sie besteht aus
• einer Matrix, bestehend aus einem Kern, umfassend
(A) 90 bis 110 Gewichtsteile Magnesium, wobei die Magnesiumquelle MgCl₂•9/2H₂O ist,
(B1) 180 bis 190 Gewichtsteile Hydroxypropylmethylcellulose,
(B2) 19,8 bis 22,2 Gewichtsteile Glycerylbehenat,
(C1) 10 bis 12 Gewichtsteile Lactose, und
(C2) 10 bis 12 Gewichtsteile kolloidales Siliciumdioxid; und
• einer Beschichtung mit
(D) 15 bis 75 Gewichtsteilen und vorzugsweise 15 bis 45 Gewichtsteilen einer Substanz, ausgewählt aus Schellack, Celluloseethern (insbesondere HPMC und HPC) und Gemischen davon.

6. Zusammensetzung gemäß Anspruch 5, **dadurch gekennzeichnet, dass** besagter Überzug
(a) eine einschichtige Beschichtung aus Schellack ist, oder
(b) eine zweischichtige Beschichtung ist, wobei jede Schicht eine Substanz umfasst, ausgewählt aus Schellack, Celluloseethern (insbesondere HPMC und HPC) und Gemischen davon.

7. Zusammensetzung gemäß Anspruch 5 oder 6, **dadurch gekennzeichnet, dass** sie *in vitro* nach 2 h in 0,1 N HCl-Medium eine Auflösungsrate (δ) des Magnesiums, das die Zusammensetzung enthält, von kleiner oder gleich 60% aufweist.

8. Zusammensetzung gemäß Anspruch 5 oder 6, **dadurch gekennzeichnet, dass** sie, mittels *in vitro* Bestimmung nach einem Auflösungssystem, umfassend zuerst die Behandlung besagter Zusammensetzung in einem 0,1 N HCl-Medium von T = 0 bis T = 2 h, anschließend Behandlung in einem Puffer bei pH 6,8 von T = 2 h bis T = 8 h, eine Auflösungsrate (δ) von Mg bezogen auf das verabreichte Mg besitzt, wie
• bei T = 2 h, δ ≤ 60%, genauer 20% ≤ δ ≤ 60% und vorzugsweise 25% ≤ δ ≤ 58% ist;
• bei T = 4 h, δ ≤ 85%, genauer 40% ≤ δ ≤ 85% und vorzugsweise 45% ≤ δ ≤ 82% ist;
• bei T = 6 h, δ ≤ 98%, genauer 60% ≤ δ ≤ 98% und vorzugsweise 80% ≤ δ ≤ 95% ist;
• bei T = 8 h, δ ≤ 100%, genauer 90% ≤ δ ≤ 100% und vorzugsweise 95% ≤ δ ≤ 99,9% ist.

9. Zusammensetzung gemäß einem der Ansprüche 5 bis 8 als Nahrungsergänzungsmittel.

10. Zusammensetzung gemäß einem der Ansprüche 5 bis 8 als Kosmetikum insbesondere für (i) die Hydratation der Haut und/oder (ii) die Behandlung oder Prävention von Hautstress.

## Claims

1. A use of a matrix for the preparation of a composition for oral administration, in tablet form, of magnesium with sustained release, said matrix having no enteric coating but having a protective coating that slows down the gastric dissolution of the Mg, and said matrix, which forms a core composed of a hydrophilic retardant (B1), a hydrophobic retardant (B2), an inert filler (C1) acting as diluent, and an inert filler (C2) acting as lubricant, being **characterized in that** it comprises, for administration of
(A) 90 to 110 parts by weight of magnesium,
the following ingredients:
(B1) 180 to 190 parts by weight of hydroxypropylmethylcellulose,
(B2) 19.8 to 22.2 parts by weight of glyceryl behenate,
(C1) 10 to 12 parts by weight of lactose, and
(C2) 10 to 12 parts by weight of colloidal silica.

2. The use according to claim 1, **characterized in that** said coating is a single-layer or multilayer film coating.

3. The use according to claim 2, **characterized in that** said coating represents 1.3% to 7.5% by weight, relative to the weight of the matrix.

4. The use according to claim 3, **characterized in that** said coating is
(a) a single-layer film coating of shellac, or
(b) a two-layer film coating, each layer comprising a substance selected from shellac, cellulose ethers (especially HPMC and HPC), and mixtures thereof.

5. A composition for oral administration, in tablet form, of magnesium with sustained release, **characterized in that** it is composed of
• a matrix constituting a core comprising
(A) 90 to 110 parts by weight of magnesium, the source of magnesium being MgCl₂.9/2H₂O,
(B1) 180 to 190 parts by weight of hydroxypropylmethylcellulose,
(B2) 19.8 to 22.2 parts by weight of glyceryl behenate,
(C1) 10 to 12 parts by weight of lactose, and
(C2) 10 to 12 parts by weight of colloidal silica; and
• a film coating of
(D) 15 to 75 parts and preferably 15 to 45 parts by weight of a substance selected from shellac, cellulose ethers (especially HPMC and HPC), and mixtures thereof.

6. The composition according to claim 5, **characterized in that** said coating is
(a) a single-layer film coating of shellac, or
(b) a two-layer film coating, each layer comprising a substance selected from shellac, cellulose ethers (especially HPMC and HPC), and mixtures thereof.

7. The composition according to claim 5 or 6, **characterized in that** *in vitro* after 2 h in a 0.1N HCl medium it exhibits a rate of dissolution (δ) of the magnesium it comprises of less than or equal to 60%.

8. The composition according to claim 5 or 6, **characterized in that**, by determination *in vitro* in accordance with a dissolution system comprising first treatment of said composition in a 0.1N HCl medium from T = 0 to T = 2 h, then treatment in a buffer at pH 6.8 from T = 2 h to T = 8 h, it has an Mg dissolution rate (δ), relative to the Mg administered, such that
• at T = 2 h, δ ≤ 60%, more specifically 20% ≤ δ ≤ 60%, and preferably 25% ≤ δ ≤ 58%;
• at T = 4 h, δ ≤ 85%, more specifically 40% ≤ δ ≤ 85%, and preferably 45% ≤ δ ≤ 82%;
• at T = 6 h, δ ≤ 98%, more specifically 60% ≤ δ ≤ 98%, and preferably 80% ≤ δ ≤ 95%; and
• at T = 8 h, δ ≤ 100%, more specifically 90% ≤ δ ≤ 100%, and preferably 95% ≤ δ ≤ 99.9%.

9. The composition according to any of claims 5 to 8, as a food supplement.

10. The composition according to any of claims 5 to 8, as a cosmetic, especially for (i) moisturizing the skin, and/or (ii) treating or preventing stress of the skin.
